# FASCICULE DE BREVET EUROPEEN

(11) **EP 0 792 419 B1**
(45) Date de publication et mention de la délivrance du brevet: **13.01.1999**
(21) Numéro de dépôt: 95940289.2
(22) Date de dépôt: 08.11.1995
(51) Int. Cl.: F16B 7/14, A61F 2/30

(54) **DISPOSITIF POUR DEPLACER DEUX CORPS L'UN PAR RAPPORT A L'AUTRE**
VORRICHTUNG ZUM VERSCHIEBEN ZWEIER KÖRPER ZUEINANDER
DEVICE FOR MUTUALLY MOVING TWO BODIES

(30) Priorité: 16.11.1994 FR 9413724
(43) Date de publication de la demande: 03.09.1997
(73) Titulaire: Soubeiran, Arnaud André, 75014 Paris (FR)
(72) Inventeur: Soubeiran, Arnaud André, 75014 Paris (FR)
(74) Mandataire: Flavenot, Bernard
(86) Numéro de dépôt international: FR9501472
(87) Numéro de publication internationale: WO9615377

(56) Documents cités:
- EP-A- 0 333 687
- EP-A- 0 358 601
- FR-A- 2 630 161
- GB-A- 2 121 922
- GB-A- 2 203 791

## Description

La présente invention concerne les dispositifs pour déplacer deux corps l'un par rapport à l'autre, qui trouvent des applications particulièrement avantageuses dans la réalisation de prothèses implantables dans le corps humain ou analogue.

Dans de nombreuses applications, il est nécessaire de déplacer deux corps l'un par rapport à l'autre tout en contrôlant ce déplacement. Lorsque les deux corps sont situés à découvert ou dans des endroits spacieux et aisément accessibles, il est relativement facile d'obtenir un tel déplacement et son contrôle, mais il n'en est pas de même lorsque les deux corps ne sont pas directement accessibles. Tel est le cas, par exemple, de deux parties d'une prothèse implantée sur un enfant que l'on souhaite pouvoir déplacer l'une par rapport à l'autre pour reproduire une croissance ou de deux parties d'un appareil destiné à corriger une déformation du corps humain en lui appliquant une contrainte que l'on veut pouvoir facilement renouveler en déplaçant les deux parties de l'appareil.

On connait déjà un dispositif, comme décrit dans le préambule de la revendication 1, permettant de déplacer deux premier et second corps l'un par rapport à l'autre. Un tel dispositif est par exemple décrit dans le EP-A-0 333 687. Ce dispositif comprend deux première et seconde pièces, des premiers moyens pour associer la première pièce au premier corps, des seconds moyens pour associer la seconde pièce au second corps, des moyens pour monter les deux première et seconde pièces en déplacement l'une part rapport à l'autre, des moyens pour appliquer une force entre les deux dites pièces, la première pièce étant en un premier matériau apte à passer d'un premier état solide indéformable à un second état au moins pâteux déformable, et réciproquement, tandis que la seconde est en un second matériau substantiellement indéformable, un élément-clapet séparant deux volumes définis par les deux pièces, cet élément étant solidaire de l'une des deux pièces et monté en coopération avec l'autre pièce de façon que, lorsque tout le dispositif est amené en température, le premier matériau fonde et s'écoule d'un volume dans l'autre pour permettre le déplacement de la première pièce par rapport à l'autre sous l'action d'un ressort.

Un tel dispositif étant amené en température dans sa totalité ne permet pas de contrôler le déplacement des deux corps l'un par rapport à l'autre. Aussi n'est-il utilisé que comme clapet dit "en tout ou rien", par exemple pour faire circuler un liquide de refroidissement dans les moteurs des véhicules automobiles.

La présente invention a ainsi pour but de réaliser un dispositif permettant de déplacer deux corps l'un par rapport à l'autre et ayant une structure qui lui permette d'être très facilement mis en oeuvre même dans des conditions d'accessibilité, d'environnement et d'encombrement sévères, qui trouve de ce fait des applications particulièrement avantageuses dans la réalisation de prothèses ou appareils implantables.

Plus précisément, la présente invention a pour objet un dispositif pour déplacer deux premier et second corps l'un par rapport à l'autre, comprenant:
- deux première et seconde pièces,
- des premiers moyens pour associer la première pièce au premier corps,
- des seconds moyens pour associer la seconde pièce au second corps,
- des moyens pour appliquer une force entre les deux dites pièces,
- une partie en saillie solidaire de l'une des deux pièces,
- une cavité réalisée dans l'autre pièce,
- la pièce dans laquelle est réalisée ladite cavité étant en un premier matériau apte à passer d'un premier état solide indéformable à un second état pâteux déformable, et réciproquement, l'état solide indéformable étant un état dans lequel ladite partie en saillie ne peut pas déformer ledit premier matériau sous l'action de ladite force et l'état pâteux déformable étant un état dans lequel ladite partie en saillie peut, sous l'action de ladite force, pénétrer dans ledit premier matériau de façon que ledit premier matériau puisse fluer autour de ladite partie en saillie, tandis que la pièce portant ladite partie en saillie est en un second matériau substantiellement indéformable,
caractérisé par le fait que ladite cavité a une forme complémentaire d'au moins une partie de ladite partie en saillie, que lesdites première et seconde pièces sont montées en déplacement l'une par rapport à l'autre de façon que ladite partie en saillie plonge dans ladite cavité, et que le dispositif comporte en outre
- des moyens pour commander le passage de la partie du premier matériau entourant ladite cavité, dudit premier état au second et réciproquement.

D'autres caractéristiques et avantages de la présente invention apparaîtront au cours de la description suivante donnée en regard des dessins annexés à titre illustratif, mais nullement limitatif, dans lesquels:

Les figures 1 et 2 représentent schématiquement un mode de réalisation d'un dispositif selon l'invention pour déplacer deux premier et second corps l'un par rapport à l'autre, respectivement dans deux états différents lors de son fonctionnement,

La figure 3 représente, vu en coupe schématique, un mode de réalisation d'un segment prothétique destiné par exemple à remplacer la partie supérieure du tibia d'un enfant, faisant application du dispositif selon l'invention,

La figure 4 représente, vu en coupe schématique, un mode de réalisation d'une tige à longueur variable pour correction chirurgicale du rachis, faisant application du dispositif selon l'invention, et

Les figures 5 et 6 représentent deux coupes longitudinales schématiques d'un mode de réalisation d'un clou médullaire utilisé pour allonger un os long tel que le fémur, faisant application du dispositif selon l'invention, la coupe représentée sur la figure 6 étant référencée VI-VI sur la figure 5 et la coupe représentée sur la figure 5 étant référencée V-V sur la figure 6.

Les six figures définies ci-dessus et annexées à la présente description représentent différents modes de réalisation d'un dispositif selon l'invention. Cependant, dans le but de faciliter la compréhension de cette description, les mêmes références y désignent les mêmes éléments, quelle que soit la figure sur laquelle elles apparaissent et quel que soit le mode de représentation de ces éléments.

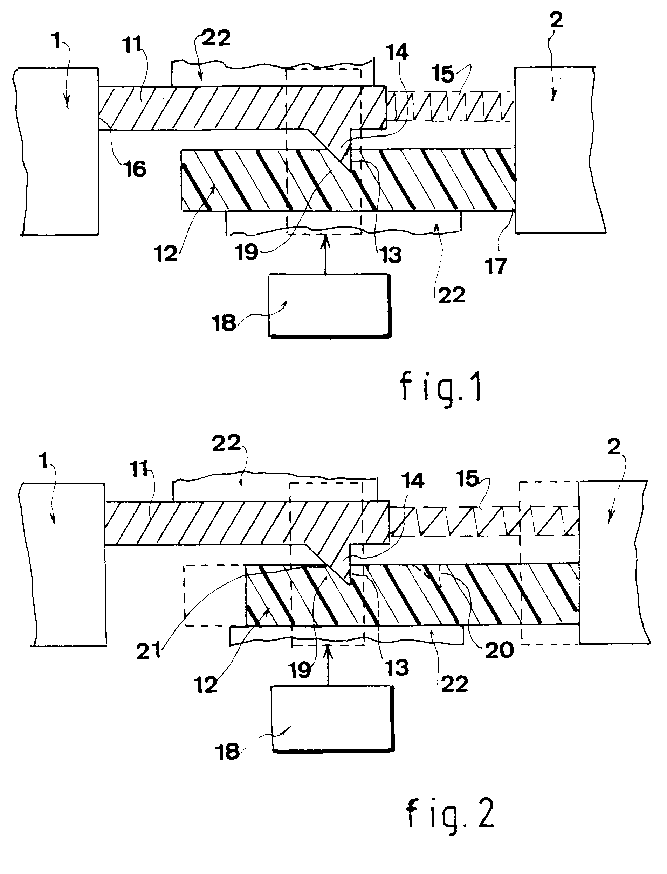

Les figures 1 et 2 représentent une forme schématique d'un mode de réalisation d'un dispositif pour déplacer deux premier 1 et second 2 corps l'un par rapport à l'autre.

Ce dispositif comporte deux première 11 et seconde 12 pièces, des premiers moyens 16 pour associer la première pièce 11 au premier corps 1 et des seconds moyens 17 pour associer la seconde pièce 12 au second corps 2. Il est bien évident que ces moyens pour associer une pièce 11, 12 à un corps 1, 2 peuvent être de tout type et que l'homme du métier saura sans aucune difficulté mettre en oeuvre ces moyens en fonction, notamment, de la nature des matériaux constituant les corps, de la nature des matériaux utilisés pour la réalisation des pièces, de la fonctionnalité des éléments constituant le dispositif, de l'application du dispositif, etc. Des exemples de réalisation de ces moyens 16 et 17 seront donnés et/ou évoqués ci-après au cours de la description de certaines applications du dispositif selon l'invention.

Des moyens 15 sont prévus pour appliquer une force entre les deux pièces 11 et 12. Cette force peut être appliquée directement entre ces deux pièces, ou indirectement par exemple comme illustré sur les figures 1 et 2 par l'intermédiaire du second corps 2. Ces moyens pour appliquer une force entre les deux pièces 11 et 12 peuvent, eux aussi, être de tout type. Ils peuvent être des moyens de traction ou de poussée, élastiques ou analogues, et prendre différentes formes. Des exemples de réalisation de ces moyens 15 seront donnés ci-après.

Une partie en saillie 14 solidaire de l'une des deux pièces, la pièce 11 dans les modes de réalisation illustrés, plonge dans une cavité 13 réalisée dans l'autre pièce 12 et complémentaire d'au moins une partie de la partie en saillie 14. Les deux pièces 11, 12 sont montées l'une par rapport à l'autre de façon qu'elles puissent se déplacer comme il sera décrit ci-après et de façon que la partie en saillie 14 plonge constamment dans la cavité aménagée dans la seconde pièce 12.

Pour faciliter et sécuriser ce déplacement relatif des deux pièces 11, 12, le dispositif comporte en outre des moyens pour guider ces deux pièces dans leur déplacement. Ces moyens de guidage ont été illustrés schématiquement en 22 et des exemples pour leur réalisation seront donnés ci-après.

La pièce 12 dans laquelle est réalisée la cavité 13 est en un premier matériau apte à passer d'un premier état solide indéformable à un second état pâteux déformable, et réciproquement. L'état solide indéformable étant un état dans lequel la partie en saillie 14 ne peut pas déformer le matériau sous l'action de la force qui est appliquée sur cette partie en saillie et l'état pâteux déformable est un état dans lequel la partie en saillie 14 peut se déplacer, ou pénétrer, dans le matériau sous l'action de cette même force, de façon que le matériau puisse fluer autour de cette partie en saillie 14. La pièce 11 de laquelle est solidaire la partie en saillie 14 est en revanche en un second matériau substantiellement indéformable.

Dans la présente description il est parfois utilisé le terme "couler" à la place de "fluer", mais il est bien entendu que ces deux termes recouvrent la même fonction en vue du même résultat.

Ce passage du premier matériau, du premier état au second, peut être obtenu par différents moyens. Parmi ces moyens, l'un des plus avantageux est constitué par le fait que le premier matériau est apte à se ramollir sous l'action d'une absorption contrôlée de calories.

Le dispositif comporte en outre des moyens pour commander le passage du premier matériau, d'un état à l'autre et réciproquement. Ces moyens de commande schématiquement représentés en 18 sur les figures 1 et 2 permettent de commander le passage, du premier état au second et réciproquement, d'au moins la partie 19 du premier matériau définissant la paroi de la cavité 13 qui se trouve devant la partie en saillie 14 par rapport au sens de déplacement, sous l'action de la force, de la pièce 11 de laquelle est solidaire la partie en saillie.

Le dispositif décrit ci-dessus fonctionne de la façon suivante:

On suppose tout d'abord que le dispositif se trouve initialement dans l'état illustré sur la figure 1. Le premier matériau est dans le premier état solide comme défini ci-dessus. Dans ce cas, la partie en saillie 14 est bloquée en butée contre la paroi rigide de la cavité 13, aussi bien à l'avant qu'à l'arrière. Les deux corps 1 et 2 sont situés à une distance donnée l'un de l'autre.

Lorsque l'on veut éloigner les deux corps 1, 2 l'un de l'autre, on commande, par les moyens 18 et pendant un temps d'une durée déterminée, le changement d'état (du premier état au second) d'au moins la partie 19 du premier matériau définie ci-avant. Le premier matériau étant alors relativement mou, la partie en saillie 14 n'est plus retenue par la paroi solide de la cavité 13. Sous l'action de la force exercée par les moyens 15, qui est supposée dans l'exemple illustré être une force élastique de poussée exercée par exemple par un ressort, les deux pièces 11, 12 s'éloignent l'une de l'autre en entraînant les deux corps 1, 2 dans leur mouvement.

Lors du déplacement relatif de la pièce 11 par rapport à la pièce 12, la partie 19 du premier matériau "flue" ou "s'écoule" autour de la partie en saillie 14 et vient combler, à l'arrière de cette partie en saillie par rapport au sens de déplacement de la pièce 11, le vide laissé dans la pièce 12 par le déplacement de la pièce 11.

Lorsque les deux pièces 11 et 12, et donc les deux corps 1 et 2, ont été éloignées l'une de l'autre d'une distance déterminée, on arrête le ramollissement du premier matériau, par exemple en commandant les moyens 18. Le premier matériau revient à son premier état solide indéformable, ce qui bloque la translation de la partie en saillie 14 contre la partie avant de la paroi de la cavité 13 qui, en quelque sorte, s'est "déplacée" de sa position origine 20 illustrée en traits interrompus sur la figure 2 à une seconde position 21. Mais, comme en plus tout le matériau de la pièce 12 entourant la partie en saillie qui plonge dans celui-ci est revenu à son état solide, la position relative des deux pièces 11 et 12 est fixée. Les deux pièces ne peuvent plus, ni s'approcher ni s'éloigner l'une de l'autre.

En commandant, par exemple de façon séquentielle, les moyens 18, on peut contrôler le passage, du premier état au second, d'au moins la partie 19 du premier matériau constituant la pièce 12. Il est ainsi possible d'obtenir l'éloignement progressif des deux corps 1 et 2 l'un par rapport à l'autre, et de contrôler cet éloignement.

Il est bien évident que l'on pourrait de la même façon contrôler le rapprochement de deux corps. Dans ce cas, la force qui s'exerce entre les deux pièces 11 et 12 serait une force de traction, et non une force de poussée comme dans l'exemple décrit ci-dessus.

La structure de dispositif décrite ci-dessus est intéressante et permet à ce dispositif d'être utilisé dans des applications très avantageuses, notamment pour la réalisation de prothèses médicales implantables dans le corps humain. Trois exemples de telles réalisations sont donnés ci-après.

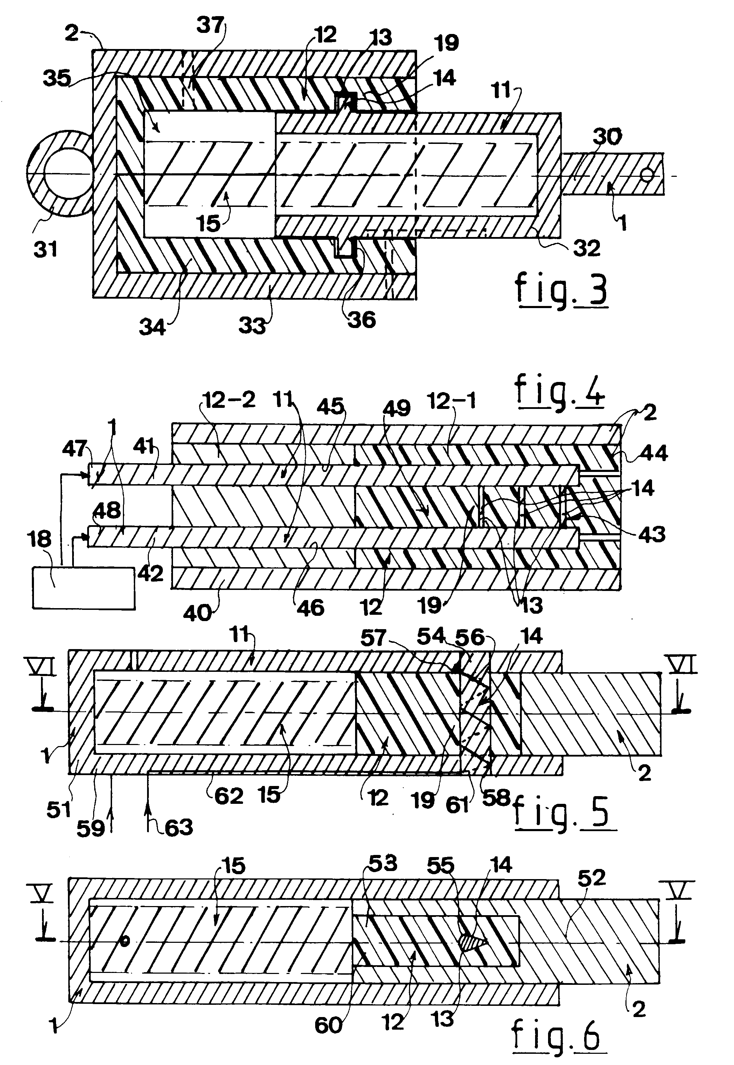

Le premier exemple illustré sur la figure 3 est relatif à un segment prothétique pour résection d'un os long, par exemple chez l'enfant. Ce segment prothétique comporte tous les éléments définis ci-avant en regard des figures 1 et 2. Dans cette application du dispositif selon l'invention, les deux corps 1 et 2 sont par exemple constitués par les extrémités 30 et 31 de deux tubes cylindriques 32 et 33, le tube 32 constituant la pièce 11 sur laquelle est réalisée la partie en saillie 14 définie ci-avant, l'extrémité 30 étant par exemple le moyen de relier le segment prothétique à l'os résiduel et l'extrémité 31 le moyen d'articuler ce segment sur une autre pièce prothétique pour constituer une articulation par exemple. Quant à la pièce 12, elle est constituée par un autre tube 34 qui comporte un logement en creux 35 dans lequel est enfiché le tube 32 à la manière d'une combinaison cylindre-piston ce qui constitue les moyens de guidage 22 définis ci-avant du déplacement du tube 32 par rapport au tube 34.

Le ressort 15 est situé dans le logement en creux 35 et exerce une force de poussée entre le fond du tube 34 et le fond du tube 32.

La partie en saillie 14 se présente sous la forme d'un anneau entourant, sur sa face extérieure, le tube 32 et plonge dans une cavité complémentaire annulaire 13 réalisée dans la paroi intérieure du tube 34. Le tube 34 est avantageusement réalisé en deux demi-coquilles pour faciliter son montage autour du tube 32.

Ce tube 34 est réalisé dans un matériau bien toléré par l'organisme, thermiquement et électriquement isolant, amagnétique, suffisamment résistant mécaniquement, en particulier à la compression, qui puisse être ramolli par chauffage à une température inférieure à celle à laquelle peuvent l'être les matériaux dans lesquels sont réalisés les autres éléments constitutifs de la prothèse, et qui retrouve en refroidissant après avoir été ramolli par chauffage, des caractéristiques proches de ses caractéristiques initiales, sinon identiques. Ce matériau est par exemple du polyéthylène.

Quant au tube 32 et à la partie en saillie 14, ils sont par exemple réalisés en une seule pièce dans une matière thermiquement et électriquement isolante, amagnétique, suffisamment résistante mécaniquement et bien tolérée par l'organisme telle que la céramique de zircone, le tube 33 pouvant être, lui aussi, réalisé en céramique de zircone.

Dans ce cas, les moyens 18 pour commander le passage d'au moins la partie 19 du premier matériau entourant la cavité 13, du premier état au second et réciproquement, sont avantageusement constitués par une couche conductrice 36 déposée sur la partie en saillie 14 pour former une boucle électrique fermée. Cette couche est obtenue par exemple par projection à la torche à plasma, sur la partie en saillie 14 qui est en contact avec la partie de premier matériau 19 définie ci-avant, d'une épaisseur de matière conductrice et bien tolérée par l'organisme telle que de l'or ou du titane. Cette boucle fermée est apte à coopérer avec un générateur de champ magnétique, comme il sera explicité ci-après, pour produire dans cette boucle un courant électrique induit. De façon connue, ce courant électrique induit échauffe par effet Joule la couche conductrice 36 qui, par conduction, va à son tour échauffer la partie de matériau 19 définie ci-avant et conduire à son ramollissement.

Le ressort 15 est constitué par exemple par un système capable d'emmagasiner et de restituer de l'énergie mécanique tel, par exemple, qu'un ressort de compression réalisé dans une matière résistante et bien tolérée par l'organisme telle que le "PHYNOX" (Marque déposée), ou encore deux ressorts contrarotatifs ou analogues.

De façon avantageuse, le logement en creux 35 est mis en communication, par exemple au moyen d'un orifice schématiquement représenté en 37, avec le milieu ambiant pour permettre aux gaz sanguins de venir compenser les variations de volume du logement en creux 35.

En outre, de façon préférentielle, le déplacement du tube 32 dans le tube 34 est guidé linéairement au moyen par exemple d'une goupille solidaire de l'un des deux tubes dont l'extrémité plonge dans une rainure réalisée dans l'autre tube. La goupille et la rainure sont représentées en traits interrompus sur la figure 3.

L'implantation d'une telle prothèse est connue des hommes du métier et ne sera donc pas décrite ici.

Le mode de réalisation du segment prothétique tel que décrit ci-dessus permet de commander par induction depuis l'extérieur de l'organisme dans lequel il a été implanté, les modifications géométriques du segment en fonction de la croissance de l'os sain symétrique.

Lorsqu'une modification de la longueur du segment prothétique qui remplace par exemple la partie supérieure du tibia d'un enfant, tel que décrit ci-dessus en regard de la figure 3, est souhaitée, il suffit de placer la partie de la jambe contenant ledit segment dans un champ magnétique orienté de préférence perpendiculairement à la boucle électrique fermée constituée par la couche conductrice 36, suffisamment intense et rapidement variable pour induire dans l'épaisseur de matière conductrice formant la boucle fermée un courant électrique et donc des pertes par effet Joule suffisantes pour provoquer, par chauffage par conduction, le ramollissement de la partie de premier matériau 19 qui borde annulairement la partie en saillie 14, mais sans arriver à la décomposition par la chaleur de ce matériau. Le tube 34 ne constitue plus alors un appui ferme pour la partie en saillie 14. Le volume ramolli 19 s'écoule ou flue autour de la partie en saillie 14, des zones de plus haute pression vers celles de plus basse pression, plus ou moins vite selon la viscosité du volume de matériau 19 ramolli et l'importance des efforts engendrés par le ressort 15 dont les caractéristiques ont été déterminées pour que ces efforts soient suffisamment supérieurs aux résistances contraires lors du déplacement de la partie en saillie 14 dans la partie 19 du premier matériau.

L'écoulement engendré se traduit par un déplacement du tube interne 32 par rapport au tube 34 dans le sens inverse de l'écoulement du volume ramolli autour de la partie en saillie 14. Le tube interne 32 reste guidé pendant son déplacement par le volume non ramolli du tube 34 et éventuellement l'extrémité de la goupille qui s'appuie dans la rainure correspondante. Le temps et l'intensité de chauffage étant contrôlés, le volume ramolli 19 reste à tout moment enclos entre des parties non ramollies, ce qui maintient la géométrie tubulaire du tube 34.

Quand la partie 19 du matériau du tube 34 est refroidi, le polyéthylène reprend sa consistance solide indéformable et le segment tibial est de nouveau capable de remplir sa fonction mécanique.

La figure 4 représente un mode de réalisation d'un appareil implantable faisant application du dispositif selon l'invention, constitué par une tige pour, par exemple, la correction chirurgicale progressive du rachis sans intervention chirurgicale intermédiaire.

Dans ce mode de réalisation selon la figure 4, la première pièce 11 est constituée par deux barres cylindriques 41, 42 montées parallèlement dans deux percées complémentaires 45, 46 réalisées dans la seconde pièce 12 et la parue en saillie 14 est constituée, dans le mode de réalisation illustré, par au moins un filament rigide 43, de préférence une pluralité, reliant les deux barres 41, 42 en passant dans des cavités 13 réalisées dans la partie 49 de la seconde pièce 12 qui est comprise entre les deux barres.

L'enfichage des deux barres cylindriques 41, 42 dans les deux percées complémentaires 45, 46 constitue dans ce mode de réalisation les moyens de guidage 22 définis ci-avant.

Les deux barres 41, 42 et les filaments 43 sont en un matériau électriquement conducteur mais la résistance électrique linéique des filaments 43 est suffisamment supérieure à la résistance électrique linéique des barres 41,42.

La seconde pièce 12 peut être réalisée en deux parties 12-1 et 12-2, comme illustré sur la figure 4. Cependant, dans ce cas, la partie 12-1 dans laquelle sont réalisées les cavités 13 peut être seule réalisée dans le premier matériau tel que défini ci-dessus, l'autre partie 12-2 étant réalisée en un matériau électriquement isolant tel que par exemple de la céramique pour obtenir une bonne rigidité totale de la tige de prothèse.

Dans cet exemple, il n'est pas spécifiquement représenté de moyens 15 pour exercer une force entre les deux barres 41, 42 et la seconde pièce 12. Ces moyens nécessaires pour obtenir le déplacement des deux corps 1 et 2 comme explicité ci-avant peuvent dans ce cas être constitués par tous moyens, par exemple des moyens externes connus de mise en traction du rachis du patient ou analogues, qui agiront respectivement sur les extrémités 47, 48 des deux barres 41, 42 et sur l'extrémité 44 de la seconde pièce 12 par l'intermédiaire des crochets qui relient de façon connue la tige au rachis.

Dans le but de rigidifier la tige, la seconde pièce 12 est avantageusement entourée d'un manchon 40 en un matériau solide comme du titane.

Dans cette réalisation, le corps 1 peut être constitué par les extrémités 47, 48 des deux barres 41, 42 et le corps 2 peut être constitué par l'extrémité 44 de la pièce 12-1 et/ou au moins une partie du manchon 40.

Pour permettre le déplacement des deux barres 41, 42 par rapport à la seconde pièce 12, on peut faire passer un courant électrique dans les deux barres et les filaments 43 délivré par un générateur de tout type schématiquement illustré qui constitue, avec les barres et les filaments, les moyens de commande 18 définis ci-avant. Le courant qui passe dans les filaments 43 est suffisamment intense pour les échauffer et ramollir la partie 19 du matériau de la seconde pièce 12, qui borde ces filaments, et pour obtenir le déplacement des barres par rapport à la seconde pièce 12 de la même façon qu'explicité précédemment.

Les figures 5 et 6 représentent deux coupes longitudinales d'un mode de réalisation d'un autre appareil implantable faisant application du dispositif selon l'invention, qui est un clou médullaire qui permet d'effectuer des allongements ou des transports osseux.

Cet appareil est destiné à être introduit par exemple dans le canal médullaire de l'os à traiter, éventuellement réalésé, et l'occupe, le plus souvent, presque entièrement. Chacune des extrémités de ce clou est rendue solidaire d'un des segments osseux que l'on veut éloigner lentement l'un de l'autre pour étirer progressivement, et avant qu'il ne se consolide, le tissu cicatriciel qui s'est formé entre les deux segments après l'ostéotomie, comme on le pratique de façon connue à l'aide de fixateurs externes. Le clou peut être allongé entre ses points de fixation aux segments osseux par déboîtement de ses parties d'un quart de millimètre à un millimètre par jour par exemple.

Cette réalisation comporte une première pièce 11 qui est constituée par un tube cylindrique creux 51 dans lequel est emmanchée la seconde pièce 12 qui est logée dans une pièce de renfort 52 qui peut constituer le second corps 2.

Cette seconde pièce 12 est constituée par un insert 53 par exemple en polyéthylène dans une fente 60 réalisée dans la pièce de renfort 52. La géométrie de cet insert est telle qu'il soit bien maintenu dans la structure de la pièce de renfort 52 qui le reçoit.

Les moyens de ressort 15 sont disposés entre le fond du tube creux 51 et la seconde pièce 12 et le corps 2.

La partie en saillie 14 est constituée par une clavette 54 qui traverse de part en part l'insert 53 dans une percée 55 qui constitue la cavité 13 définie ci-avant. Cette clavette est constituée par exemple d'un noyau de matière de haute résistance mécanique, telle que le "PHYNOX", isolé électriquement et thermiquement par une couche d'une autre matière suffisamment résistante mécaniquement et isolante telle que la céramique de zircone, par exemple projetée à la torche à plasma.

Un fil fin de matière conductrice 56 telle que le "PHYNOX" (0,1 millimètre à 0,3 millimètre de diamètre) est bobiné sur cette couche isolante éventuellement usinée de façon à présenter une gorge en forme de spirale qui permet d'éviter le contact entre deux spires de fil. Une extrémité 57 de ce fil est soudée, par exemple au moyen d'un laser, sur le tube 51 et l'autre extrémité 58 reliée à une première extrémité 61 d'un conducteur électrique 62 tel qu'un fil d'or isolé, par exemple, par une gaine de polytétrafluoroéthylène, et qui court dans une gorge pratiquée à l'extérieur du tube 51 jusqu'à l'extrémité fermée 59 de ce tube.

C'est dans le fil bobiné sur la couche isolante de la clavette que sont provoquées les pertes par effet Joule suffisantes pour ramollir la partie 19 du premier matériau bordant la clavette 54. La clavette peut être profilée de façon à faciliter son déplacement dans le sens souhaité au sein du premier matériau quand il est ramolli.

Le tube 51 et la seconde extrémité 63 du conducteur électrique 62 sont par exemple connectés à une boucle électrique, non représentée, qui est implantée dans le corps humain, par exemple en position sous-cutanée. Cette boucle peut être soumise à l'action d'un champ magnétique rapidement variable pour induire dans la boucle un courant électrique et alimenter le fil fin 56 qui devient alors le siège de l'échauffement par effet Joule mentionné ci-dessus.

Le fonctionnement du clou illustré sur les figures 5 et 6 se déduit très facilement du fonctionnement des autres prothèses décrites ci-avant et ne sera donc pas plus amplement décrit ici.

Dans la description ci-dessus, il a été donné quelques exemples particuliers de réalisation des moyens de guidage 22. Quant aux moyens 16 et 17 pour associer les pièces 11, 12 aux corps 1, 2, ils peuvent prendre différentes formes. Par exemple, dans le mode de réalisation selon la figure 3, les moyens 17 sont constitués d'un emmanchement du tube 34 dans le tube 33, le tube 34 étant en outre solidarisé au tube 33 par tout moyen tel que collage, clavette, écrou ou analogue. Dans ce même mode de réalisation, les moyens 16 sont constitués par au moins une partie de la pièce 11 elle-même ou une pièce annexe solidarisée à la pièce 11 par tous moyens, soudage, réalisation dans la masse, etc. Ces moyens 16 et 17 ne sont pas précisés pour les réalisations illustrées sur la figure 4 et les figures 5 et 6, mais l'homme du métier saura les mettre en oeuvre sans aucune difficulté.

Dans les exemples décrits ci-dessus, le déplacement des deux corps l'un par rapport à l'autre est une translation linéaire. Il est cependant bien évident que l'invention peut s'appliquer à toute autre sorte de mouvement, rotation, torsion ou toute combinaison de ces mouvements avec une translation.

## Revendications

1. Dispositif pour déplacer deux premier et second corps (1, 2) l'un par rapport à l'autre, comprenant:
- deux première et seconde pièces (11, 12),
- des premiers moyens (16) pour associer la première pièce au premier corps,
- des seconds moyens (17) pour associer la seconde pièce au second corps,
- des moyens (15) pour appliquer une force entre les deux dites pièces,
- une partie en saillie (14) solidaire de l'une des deux pièces (11),
- une cavité (13) réalisée dans l'autre pièce (12),
- la pièce (12) dans laquelle est réalisée ladite cavité (13) étant en un premier matériau apte à passer d'un premier état solide indéformable à un second état pâteux déformable, et réciproquement, l'état solide indéformable étant un état dans lequel ladite partie en saillie ne peut pas déformer ledit premier matériau sous l'action de ladite force et l'état pâteux déformable étant un état dans lequel ladite partie en saillie peut, sous l'action de ladite force, pénétrer dans ledit premier matériau de façon que ledit premier matériau puisse fluer autour de ladite partie en saillie, tandis que la pièce (11) portant ladite partie en saillie (14) est en un second matériau substantiellement indéformable,
caractérisé par le fait que ladite cavité a une forme complémentaire d'au moins une partie de ladite partie en saillie,
que lesdites première et seconde pièces sont montées en déplacement l'une par rapport à l'autre de façon que ladite partie en saillie plonge dans ladite cavité, et que le dispositif comporte en outre
- des moyens pour commander le passage de la partie (19) du premier matériau entourant ladite cavité (15), dudit premier état au second et réciproquement.

2. Dispositif selon la revendication 1, caractérisé par le fait qu'il comporte en outre des moyens (22) pour guider les deux dites première et seconde pièces (11, 12) dans leur déplacement.

3. Dispositif selon l'une des revendications 1 et 2, caractérisé par le fait que les moyens pour commander le passage d'au moins la partie (19) du premier matériau entourant ladite cavité (13), dudit premier état au second et réciproquement, sont des moyens pour ramollir, sous l'action d'une absorption contrôlée de calories, au moins la partie du premier matériau définissant la paroi de ladite cavité (13) se trouvant devant la partie en saillie (14) par rapport au sens de déplacement de la pièce (11) de laquelle est solidaire ladite partie en saillie.

4. Dispositif selon l'une des revendications 1 à 3, caractérisé par le fait que ladite première pièce (11) est constituée par un premier tube (32) et que ladite seconde pièce (12) est constituée par un deuxième tube (34) comportant un premier logement en creux (35), ledit premier tube (32) étant enfiché dans cedit premier logement en creux.

5. Dispositif selon la revendication 4, caractérisé par le fait que lesdits moyens pour appliquer une force entre les deux dites pièces sont constitués par un ressort (15) situé dans ledit premier logement en creux (35) entre les fonds respectivement des premier et deuxième tubes (32, 34).

6. Dispositif selon l'une des revendications 4 et 5, caractérisé par le fait que ladite partie en saillie (14) présente la forme d'un anneau entourant, sur sa face extérieure, ledit premier tube (32) et plonge dans ladite cavité (13) affectant une forme annulaire réalisée dans la paroi intérieure dudit deuxième tube (34).

7. Dispositif selon la revendication 6, caractérisé par le fait que lesdits moyens (18) pour commander le passage d'au moins la partie (19) du premier matériau entourant ladite cavité (13), du premier état au second et réciproquement, comportent une couche conductrice (36) déposée sur ladite partie en saillie annulaire (14) pour former une boucle électrique fermée apte à coopérer avec un générateur de champ magnétique.

8. Dispositif selon l'une des revendications 1 à 3, caractérisé par le fait que ladite première pièce (11) est constituée par deux barres cylindriques (41, 42) montées parallèlement dans deux percées complémentaires (45, 46) réalisées dans ladite seconde pièce (12), la partie en saillie (14) étant constituée par au moins un filament rigide (43) reliant les deux dites barres (41,42).

9. Dispositif selon la revendication 8, caractérisé par le fait que les moyens (18) pour commander le passage d'au moins la partie du premier matériau entourant ladite cavité, dudit premier état au second et réciproquement, comportent au moins un générateur de courant électrique relié aux deux barres (41, 42), lesdites barres (41, 42) et ledit filament rigide (43) étant en un matériau électriquement conducteur, la résistance électrique linéique dudit filament (43) étant supérieure à la résistance électrique linéique desdites barres (41, 42), ledit premier matériau dans lequel est réalisée ladite seconde pièce (12) étant en un matériau non électriquement conducteur.

10. Dispositif selon l'une des revendications 1 à 3, caractérisé par le fait que ladite première pièce (11) est constituée par un troisième tube cylindrique creux (51), ladite seconde pièce (12) étant emmanchée dans cedit troisième tube (51).

11. Dispositif selon la revendication 10, caractérisé par le fait que ladite seconde pièce (12) est constituée par un insert (53) situé dans une fente (60) réalisée dans une pièce de renfort (52).

12. Dispositif selon la revendication 11, caractérisé par le fait que ladite partie en saillie (14) est constituée par au moins une clavette (54) traversant de part en part ledit insert (53).

13. Dispositif selon la revendication 12, caractérisé par le fait que ladite clavette (54) est constituée d'un noyau de matière de haute résistance mécanique électriquement et thermiquement isolé par une couche de matière résistante mécaniquement et isolante.

14. Dispositif selon la revendication 13, caractérisé par le fait que les moyens pour commander le passage d'au moins la partie du premier matériau entourant ladite cavité, dudit premier état au second et réciproquement, comportent au moins un fil fin de matière conductrice (56) bobiné sur ladite clavette (54), un générateur de courant électrique, et des moyens pour coupler ledit générateur audit fil fin (56).

## Patentansprüche

1. Vorrichtung zum Verschieben zweier, eines ersten und eines zweiten, Körpers (1, 2) zueinander, umfassend
- zwei, ein erstes und ein zweites, Teile (11, 12),
- erste Mittel (16) zum Verbinden des ersten Teils mit dem erten Körper,
- zweite Mittel (17) zum Verbinden des zweiten Teils mit dem zweiten Körper,
- Mittel (15) zum Anlegen einer Kraft zwischen den beiden Teilen,
- einen vorspringenden Abschnitt (14), der mit einem der Teile (11) fest verbunden ist,
- eine Ausnehmung (13), die in dem anderen Teil (12) realisiert ist,
- wobei das Teil (12), in dem die Ausnehmung (13) realisiert ist, aus einem ersten Material ist, das geeignet ist, von einem festen, nicht deformierbaren ersten Zustand in einen deformierbaren pastigen Zustand und umgekehrt überzugehen, wobei der undeformierbare feste Zustand ein Zustand ist, in dem der vorspringende Abschnitt unter der Wirkung der Kraft das erste Material nicht deformieren kann, und der deformierbare pastige Zustand ein Zustand ist, in dem der vorspringende Abschnitt unter der Wirkung der Kraft in das erste Material derart eindringen kann, daß das erste Material um den vorspringenden Abschnitt fließen kann, während das Teil (11), das den vorspringenden Abschnitt (14) trägt, aus einem zweiten, im wesentlichen nicht deformierbaren Material ist,
- daß die Ausnehmung eine komplementäre Form zu wenigstens einem Teil des vorspringenden Abschnitts aufweist,
- daß das erste und zweite Teil derart in bezug zueinander verschiebbar angeordnet sind, daß der vorspringende Abschnitt in die Ausnehmung eintaucht, und daß die Vorrichtung dadurch gekennzeichnet ist, daß sie zudem
- Mittel zum Steuern des Übergangs des Abschnitts (19) des ersten, die Ausnehmung (15) umgebenden Materials vom ersten Zustand in den zweiten und umgekehrt aufweist.

2. Vorrichtung nach Anspruch 1, dadurch gekennzeichnet, daß sie zudem Mittel (22) zum Führen der beiden, des ersten und zweiten, Teile (11, 12) bei ihrer Verschiebung umfaßt.

3. Vorrichtung nach einem der Ansprüche 1 und 2, dadurch gekennzeichnet, daß die Mittel zum Steuern des Übergangs wenigstens einen Abschnitt (19) des ersten, die Ausnehmung (13) umgebenden Materials vom ersten Zustand in den zweiten und umgekehrt, Mittel zum Erweichen unter der Wirkung einer kontrollierten Kalorienabsorption wenigstens des Teils des ersten Materials sind, das die Wandung der Ausnehmung (13) bildet, die sich vor dem vorspringenden Abschnitt (14) in bezug auf die Verschiebungsrichtung des Teils (11) befindet, mit dem der vorspringende Abschnitt fest verbunden ist.

4. Vorrichtung nach einem der Ansprüche 1 bis 3, dadurch gekennzeichnet, daß das erste Teil (11) aus einem ersten Rohr (32) und das zweite Teil (12) aus einem zweiten Rohr (34) gebildet wird, das einen ersten hohlen Aufnahmeraum (35) umfaßt, wobei das erste Rohr (32) in diesen ersten hohlen Aufnahmeraum eingesteckt ist.

5. Vorrichtung nach Anspruch 4, dadurch gekennzeichnet, daß die Mittel zum Anlegen einer Kraft zwischen den beiden Teilen durch eine Feder (15) gebildet werden, die in dem ersten hohlen Aufnahmeraum (35) zwischen den Böden des ersten und zweiten Rohrs (32, 34) angeordnet ist.

6. Vorrichtung nach einem der Ansprüche 4 und 5, dadurch gekennzeichnet, daß der vorspringende Abschnitt (14) die Form eines Rings aufweist, der das erste Rohr (32) auf seiner Außenseite umgibt und in die Ausnehmung (13) taucht, der eine Ringform zugewiesen ist, die in der Innenwand des zweiten Rohrs (34) realisiert ist.

7. Vorrichtung nach Anspruch 6, dadurch gekennzeichnet, daß die Mittel (18) zum Steuern des Übergangs wenigstens des Abschnitts (19) des ersten, die Ausnehmung (13) umgebenden Materials vom ersten Zustand in den zweiten und umgekehrt eine leitende Schicht (36) umfassen, die auf dem ringförmigen vorspringenden Abschnitt (14) angeordnet ist, um eine geschlossene elektrische Schleife zu bilden, die geeignet ist, mit einem Magnetfeldgenerator zusammenzuarbeiten.

8. Vorrichtung nach einem der Ansprüche 1 bis 3, dadurch gekennzeichnet, daß das erste Teil (11) durch zwei zylindrische Stäbe (41, 42) gebildet wird, die parallel in zwei komplementären Löchern (45, 46), die in dem zweiten Teil (12) realisiert sind, montiert sind, wobei der vorspringende Abschnitt (14) durch wenigstens ein starres Filament (43) gebildet wird, das die beiden Stäbe (41, 42) miteinander verbindet.

9. Vorrichtung nach Anspruch 8, dadurch gekennzeichnet, daß die Mittel (18) zum Steuern des Übergangs wenigstens des Abschnitts des die Ausnehmung umgebenden ersten Materials vom ersten in den zweiten Zustand und umgekehrt wenigstens einen elektrischen Stromgenerator umfasen, der mit den beiden Stäben (41, 42) verbunden ist, wobei die Stäbe (41, 42) und das starre Filament (43) aus einem elektrisch leitenden Material bestehen, wobei der spezifische elektrische Widerstand des Filaments (43) größer als derjenige der Stäbe (41, 42) ist, wobei das erste Material, aus dem das zweite Teil (12) realisiert ist, aus einem elektrisch nicht leitenden Material ist.

10. Vorrichtung nach einem der Ansprüche 1 bis 3, dadurch gekennzeichnet, daß das erste Teil (11) aus einem dritten zylindrischen, hohlen Rohr (51) gebildet ist, wobei das zweite Teil (12) in dem dritten Rohr (51) gelagert ist.

11. Vorrichtung nach Anspruch 10, dadurch gekennzeichnet, daß das zweite Teil (12) durch einen Einsatz (53) gebildet ist, der in einem Schlitz (60) angeordnet ist, der in einem Verstärkungsteil (52) realisiert ist.

12. Vorrichtung nach Anspruch 11, dadurch gekennzeichnet, daß der vorspringende Abschnitt (14) durch wenigstens einen Keil (54) gebildet ist, der sich durch den Einsatz (53) hindurch erstreckt.

13. Vorrichtung nach Anspruch 12, dadurch gekennzeichnet, daß der Keil (54) aus einem Kern aus mechanisch hoch widerstandsfähigem Material gebildet ist, das durch eine Schicht aus mechanisch restistentem und isolierendem Material elektrisch und thermisch isoliert ist.

14. Vorrichtung nach Anspruch 13, dadurch gekennzeichnet, daß die Mittel zum Steuern des Übergangs wenigstens des Teils des ersten, die Ausnehmung umgebenden Materials vom ersten Zustand in den zweiten und umgekehrt wenigstens einen feinen Draht aus leitendem Material (56), der auf den Keil (54) gewickelt ist, einen elektrischen Stromgenerator und Mittel zum Koppeln des Generators an den feinen Draht (56) umfasen.

## Claims

1. A device for displacing two first and second bodies (1, 2) relative to each other, comprising:
- two first and second pieces (11, 12),
- first means (16) for associating the first piece with the first body,
- second means (17) for associating the second piece with the second body,
- means (15) for applying a force between said two pieces,
- a projecting portion (14) secured to one of the two pieces (11),
- a cavity (13) formed in the other piece (12),
- the piece (12) in which said cavity (13) is formed being made of a first material suitable for passing from an undeformable hard first state to a deformable soft second state, and vice versa, the undeformable hard state being a state in which said projecting portion cannot deform said first material under drive from said force, and the deformable soft state being a state in which said projecting portion, under drive from said force, can penetrate into said first material in such a manner that said first material can creep around said projecting portion, while the piece (11) carrying said projecting portion (14) is made of a second material that is substantially undeformable,
characterized by the fact that said cavity is complementary in shape to at least a part of said projecting portion, that said first and second pieces are mounted for displacement relative to each other in such a manner that said projecting portion engages in said cavity, and that the device further includes means for controlling the passage of the portion (19) of the first material that surrounds the cavity (13) from the first state to the second state, and vice versa.

2. A device according to claim 1, characterized by the fact that it further includes means (22) for guiding said two first and second pieces (11, 12) in their displacement.

3. A device according to one of claims 1 and 2, characterized by the fact that the means for controlling the passage of at least the portion (19) of the first material that surrounds said cavity (13), from the first state to the second state and vice versa, are means for softening at least the portion of the first material which defines the wall of said cavity (13) lying in front of the projecting portion (14) relative to the displacement direction of the piece (11) to which said projecting portion is secured, said softening being obtained by the action of controlled heat absorption.

4. A device according to one of claims 1 to 3, characterized by the fact that said first piece (11) is constituted by a first tube (32) and that said second piece (12) is constituted by a second tube (34) having a first hollow housing (35), said first tube (32) being engaged in said first hollow housing.

5. A device according to claim 4, characterized by the fact that said means for applying a force between said two pieces are constituted by a spring (15) situated in said first hollow housing (35) between the respective ends of the first and second tubes (32, 34).

6. A device according to one of claims 4 and 5, characterized by the fact that said projecting portion (14) is in the form of a ring surrounding said first tube (32) on the outside face thereof and engages in said cavity (13) which is in the form of an annulus formed in the inside wall of said second tube (34).

7. A device according to claim 6, characterized by the fact that said means (18) for controlling the passage of at least the portion (19) of the first material that surrounds the cavity (13), from the first state to the second state and vice versa, include a conductive layer (36) deposited on said annular projecting portion (14) to form a closed electric loop suitable for co-operating with a magnetic field source.

8. A device according to one of claims 1 to 3, characterized by the fact that said first piece (11) is constituted by two cylindrical bars (41, 42) mounted in parallel in two complementary bores (45, 46) formed in said second piece (12), the projecting portion (14) being constituted by at least one rigid filament (43) interconnecting said two bars (41, 42).

9. A device according to claim 8, characterized by the fact that the means (18) for controlling the passage of at least the portion (19) of the first material that surrounds the cavity (13), from the first state to the second state and vice versa, comprise at least one electric current source connected to the two bars (41, 42), said bars (41, 42) and said rigid filament (43) being of an electrically conductive material, the electrical resistance per unit length of said filament (43) being greater than the electrical resistance per unit length of said bars (41, 42), said first material from which said second piece (12) is made being a material that is not electrically conductive.

10. A device according to any one of claims 1 to 3, characterized by the fact that said first piece (11) is constituted by a third hollow cylindrical tube (51), said second piece (12) being engaged in said third tube (51).

11. A device according to claim 10, characterized by the fact that said second piece (12) is constituted by an insert (53) situated in a slot (60) formed in a reinforced piece (52).

12. A device according to claim 11, characterized by the fact that said projecting portion (14) is constituted by at least one key (54) passing right through said insert (53).

13. A device according to claim 12, characterized by the fact that said key (54) is constituted by a core of mechanically strong material that is electrically and thermally insulated by a layer of mechanically strong and isolating material.

14. A device according to claim 13, characterized by the fact that the means for controlling the passage of at least the portion of the first material that surrounds the cavity, from the first state to the second state and vice versa, comprise at least one fine wire of conductive material (56) wound on said key (54), an electric current source, and means for coupling said source to said fine wire (56).
